# EUROPÄISCHE PATENTSCHRIFT

(11) **EP 0 545 960 B1**
(45) Veröffentlichungstag und Bekanntmachung des Hinweises auf die Patenterteilung: **06.09.1995**
(21) Anmeldenummer: 91914470.9
(22) Anmeldetag: 13.08.1991
(51) Int. Cl.: A61M 15/00

(54) **TREIBGASFREIES INHALATIONSGERÄT**
INHALATION DEVICE WITHOUT PROPELLANT GAS
INHALATEUR SANS GAZ PROPULSEUR

(30) Priorität: 30.08.1990 DE 4027390
(43) Veröffentlichungstag der Anmeldung: 16.06.1993
(73) Patentinhaber: BOEHRINGER INGELHEIM KG, 55216 Ingelheim (DE)
(72) Erfinder: POSS, Gerhard, D-6905 Schriesheim (DE); WITTEKIND, Jürgen, D-6000 Frankfurt/Main 70 (DE)
(74) Vertreter: Fuchs Mehler Weiss
(86) Internationale Anmeldenummer: EP9101531
(87) Internationale Veröffentlichungsnummer: WO9204067

(56) Entgegenhaltungen:
- EP-A- 0 407 028
- DE-C- 25 422
- DE-C- 332 767
- FR-A- 544 945

## Beschreibung

Die Erfindung bezieht sich auf ein treibgasfreies Inhalationsgerät, mit einem Vorrat an zu inhalierender medizinischer Substanz, einer manuell betätigbaren Dosiereinrichtung zur Entnahme einer vorgegebenen Dosis der medizinischen Substanz für den jeweiligen Inhalationsvorgang, mit einem Mundstück zum aktiven Einatmen sowie einem Luftraum zur Verteilung der jeweiligen Dosis der medizinischen Substanz im Luftstrom u. einer Halterung, in welcher der Vorrat an medizinischer Substanz aufgenommen ist.

Ein derartiges Inhalationsgerät ist aus der DE-A-332767 bekanntgeworden.

Bei einem bekannten Inhalationsgerät gemäß der DE-A-3535561 ist unterhalb des Vorratsbehälters und parallel zum Mundstück eine Drehschleuse vorgesehen, die Vertiefungen (Dosierkammern) zum Bemessen der medizinischen Substanz aufweist. Sind die Vertiefungen jeweils dem Vorratsbehälter zugewendet, werden sie selbsttätig befüllt. Wird durch eine 180°-Verdrehung der Drehschleuse die befüllte Vertiefung der Luftkammer des Mundstückes zugewendet, fällt die Pulverdosis durch die Schwerkraft, unterstützt durch einen Rüttelmechanismus, aus der Vertiefung in einen Hohlraum des Luftkanals und wird von dort aus mit Hilfe aktiver Einatmung in die Lungen bzw. Bronchien des Patienten inhaliert. Dabei weist der Luftkanal einen Drosselbereich auf, der das Mischen der Luft mit der medizinischen Substanz durch Turbulenz fördern soll.

Dieses bekannte Gerät beruht - wie zahlreiche andere bekannte Geräte - auf dem Prinzip, daß in einem Vorratsbehälter die medizinische Substanz in pulverförmigem Zustand bereitgehalten wird und die Dosis durch Befüllen einer Dosierkammer bemessen wird. Der Inhalt der Dosierkammer wird dann unter Ausnützung der Schwerkraft bzw. mit Hilfe der aktiven Einatmung des Patienten, ggf. mechanisch unterstützt, ausgetragen.

Ein Inhalationsgerät nach diesem Dosierprinzip weist den Nachteil auf, daß die zu inhalierende Dosis nicht ausreichend reproduzierbar ist und nicht befriedigend dispergiert wird.

Der gleiche Nachteil haftet einem Inhalationsgerät gemäß der gattungsbildenden DE-A-332767 an. Gegenüber dem Gerät gemäß der oben erwähnten Druckschrift weist das gattungsgemäße Gerät noch eine Halterung zur Aufnahme der medizinischen, zu inhalierenden Substanz auf. Ein Schlagwerk mit Schlägern sorgt bei manueller Betätigung für ein Vermischen der medizinischen Substanz mit der einzuatmenden Luft.

Die so zur Inhalation vorbereitete Dosis ist nicht in hinreichendem Maße reproduzierbar. So ist allein schon die Menge der Substanz abhängig davon, mit welcher Kraft das Schlagwerk betätigt wird. So ist es einsichtig, daß bei entsprechend kräftiger Betätigung die Dosis der medizinischen Substanz entsprechend höher ausfällt als wenn das Schlagwerk nur relativ sanft betätigt wird.

Im übrigen ist ein weiteres Inhalationsgerät bekannt geworden aus der nachveröffentlichten EP-A- 0 407 028. In dem darin beschriebenen, recht kompakten Gerät ist eine medizinische Substanz in einer vorgegebenen geometrischen Struktur verfestigt.

Eine manuell betätigbare Dosierungvorrichtung ist darin als eine Abriebanordnung aus einem drehbaren, wendelförmigen Schaber mit einer Blattkante beschrieben.

Der Erfindung liegt die Aufgabe zugrunde, ausgehend von dem eingangs bezeichneten treibgasfreien Inhalationsgerät dieses so zu gestalten, daß die eingeatmete Dosis im hohen Maße reproduzierbar ist und gut dispergiert wird.

Die Lösung dieser Aufgabe gelingt gemäß der Erfindung dadurch, daß die medizinische Substanz verfestigt in einer vorgegebenen geometrischen Struktur vorliegt und daß ihr als Dosiereinrichtung eine Abriebanordnung in Form einer drehbaren Bürstenanordnung zum mechanischen Abrieb der vorgegebenen Dosis zugeordnet ist.

Bei dem erfindungsgemäßen treibgasfreien Inhalationsgerät wird daher die medizinische Substanz nicht in Pulverform sondern in fester Form vorrätig gehalten; sie wird erst unmittelbar vor dem jeweiligen Inhalationsvorgang durch einen mechanischen Vorgang für die benötigte Dosis pulverisiert. Dieser mechanische Vorgang, d.h. der Abrieb der medizinischen Substanz, kann verhältnismäßig genau und reproduzierbar gestaltet werden, so daß auch die eingeatmete Dosis an pulverisierter medizinischer Substanz im hohen Maße reproduzierbar ist.

Der verfestigte Vorrat der medizinischen Substanz kann beispielsweise hergestellt werden, indem man mikronisierten Wirkstoff (Teilchengröße < 10 µm) mit einem physiologisch unbedenklichen Hilfs- oder Trägerstoff, beispielsweise Lactose, mischt und zu einem Körper verpreßt, der in Richtung seiner Längsachse einen konstanten Querschnitt hat. Die Form des Querschnitts kann im übrigen vielfältig variiert werden, so daß der Körper z.B. ein Quader, ein Zylinder oder ein Prisma sein kann. Um zu erreichen, daß der Abrieb möglichst gleichmäßig erfolgt, kann eine Vorrichtung vorgesehen sein, die eine Drehung des Körpers um seine Längsachse bewirkt, etwa in der Weise, daß der Körper bei jeder Betätigung des Geräts um 25 - 90°, beispielsweise jeweils 55°, gedreht wird.

Anhand von in den Zeichnungen dargestellten Ausführungsbeispielen wird die Erfindung näher erläutert. Dabei ergeben sich auch ausgestaltende Merkmale der Erfindung.

Es zeigen:
- Fig. 1: ein Ausführungsbeispiel des erfindungsgemäßen treibgasfreien Inhalationsgerätes im Nichtgebrauchszustand,
- Fig. 2: das Inhalationsgerät nach Fig. 1 in gespanntem Zustand, d.h. bereit zur Inhalation,
- Fig. 3: einen Schnitt entlang der Linie A-A in Fig. 1 unter Darstellung einer anderen Ausführungsform.

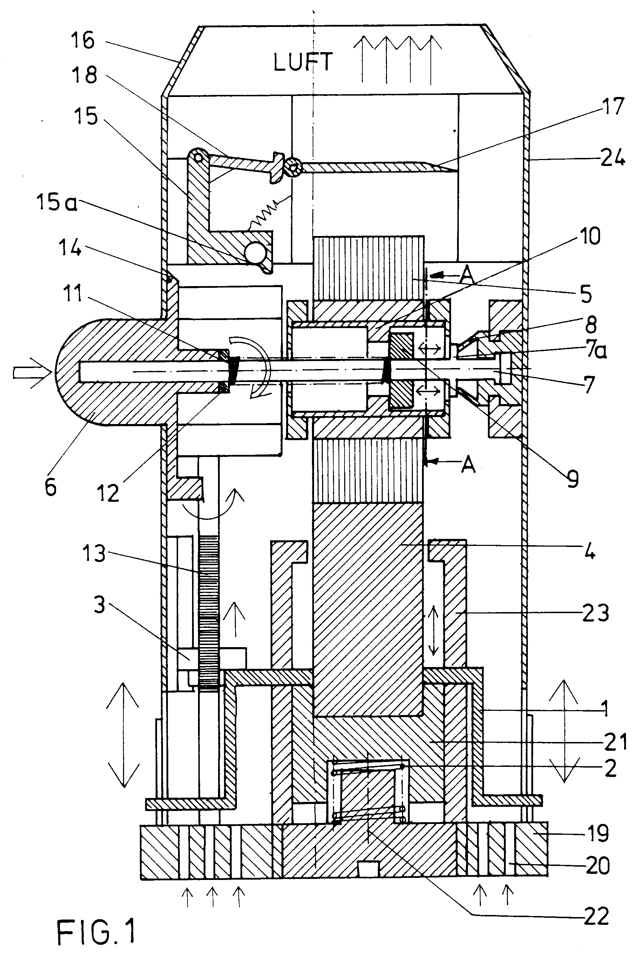

Das in Fig. 1 dargestellte treibgasfreie Inhalationsgerät besteht aus einem Gehäuse 24, welches kopfseitig ein Mundstück 16 besitzt und welches am anderen Ende durch eine Bodenplatte 19 abgeschlossen ist, die Lufteintrittschlitze 20 und ein zentrales, herausschraubbares Bodenstück 22 besitzt.

Im Gehäuse ist im Bereich des bodenseitigen Endes ein Tablettenhalter 1 axial verschiebbar angeordnet, an dem eine quaderförmige Tablette 4 der zu dosierenden und inhalierenden medizinischen Substanz in einem Tablettensockel 21 befestigt ist. Die Bewegung der Tablette 4 wird in einer Tablettenführung 23 geführt.

Das Inhalationsgerät weist ferner eine drehbare Bürstenanordnung 5 auf, die in Kontakt mit der Tablette 4 bringbar ist. Durch eine nicht dargestellte, auf das Inhalationsgerät aufgestülpte Schutzkappe wird im Ruhezustand der Tablettenhalter 1 unter Spannen einer Druckfeder 2, die sich am zentralen Bodenstück 22 und am Tablettensockel 21 abstützt, niedergehalten. Beim Entfernen der Schutzkappe wird der Tablettenhalter 1 durch die Druckfeder 2 gegen einen Anschlag 3 und damit die im Tablettenhalter 1 befestigte Tablette 4 gegen die Bürste 5 gedrückt.

Im oberen Teil des Inhalationsgerätes ist eine Drucktaste 6 vorgesehen, der über ein Schubglied 12 eine in Querrichtung verlaufende Spindel 7 zugeordnet ist. Diese Spindel stützt sich mit dem Anschlag 7a gegen eine Tellerfeder 8 ab. Mit der Spindel 7 ist ein Antriebs-Reibrad 9 fest verbunden, welches mit einem der Bürste 5 zugeordneten, sie antreibenden Bürstenreibrad 10 in Wirkeingriff steht. Zwischen Drucktaste 6 und Antriebs-Reibrad 9 ist eine Feder 11 angeordnet.

Mit der Betätigung der Drucktaste 6 wird das Inhalationsgerät gespannt, d.h. in den funktionsbereiten Zustand gebracht. Die Anordnung der Elemente ist dabei so getroffen, daß die Spindel 7 zunächst eine geringfügige Translationsbewegung nach rechts gegen die Tellerfeder 8 ausführt. In Figur 1 ist dies auf Höhe des Schnittes A-A durch entsprechende Pfeile angedeutet. Dabei wird das mit der Spindel 7 fest verbundene Antriebs-Reibrad 9 vom Bürstenreibrad 10 getrennt. Beim weiteren Eindrücken der Drucktaste 6 wird die Feder 11 gespannt und die Spindel 7 führt dabei eine bis mehrere Drehungen aus, bewirkt durch ein steiles Schraubengewinde auf der Spindel und das fest mit der Drucktaste 6 verbundene Schubglied 12.

Zur Verstellung des Anschlages 3 ist eine weitere Spindel 13 vorgesehen, die parallel zur Gerätelängsachse angeordnet ist. Diese Spindel ist über bekannte Bauelemente derartig mit der anderen Spindel 7 gekoppelt, daß gleichzeitig beim Eindrücken der Drucktaste durch form- oder kraftschlüssigen Eingriff die Spindel 13 um einen definierten Winkel, z.B. 180°, gedreht wird. Dies bewirkt, daß sich der Anschlag 3 um einen definierten Hub weiterbewegt. Hierdurch wird die von der Tablette abzubürstende Menge (Dosis) festgelegt und die Dosierkonstanz gewährleistet.

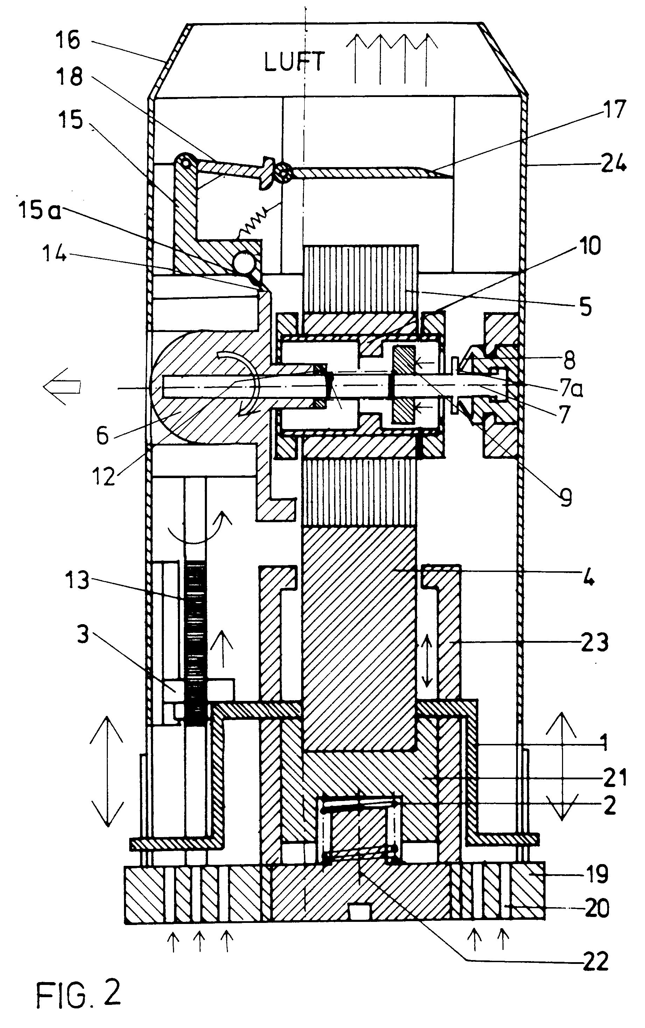

Zur Arretierung des gespannten Zustandes weist die Drucktaste einen federnden Klinkenzapfen 14 auf, der mit einer Klinkennase 15a eines Winkelhebels 15 in Eingriff bringbar ist. Ist die Drucktaste 6 vollständig eingedrückt, wird sie mittels des federnden Zapfens 14 am Winkelhebel 15 arretiert. Die Feder 11 ist nun vollständig gespannt. Dieser Zustand ist in Fig. 2 dargestellt.

Zum selbsttätigen Auslösen des gespannten Zustandes bei aktivem Einatmen in der Phase des Inhalierens ist eine federvorgespannte Klappe 17 und ein Kniehebelmechanismus 18 vorgesehen. Beim Inhalieren durch das Mundstück 16 entsteht ein Unterdruck vor der Klappe 17, so daß diese nach oben schwenkt und dabei über den Kniehebelmechanismus 18 und den Winkelhebel 15 die Drucktaste 6 entriegelt. Diese wird durch die vorgespannte Feder 11 in die Ausgangsstellung nach Fig. 1 zurückbewegt, wobei das Schraubengewinde auf der Spindel 7 und das Schubglied 12 an der Drucktaste 6 eine Rotation der Spindel 7 bewirkt. Das Antriebs-Reibrad 9 wird durch die Tellerfeder 8 an das Bürstenreibrad 10 angepreßt, so daß die Bürste ebenfalls rotiert und dabei Pulver in der gewünschten Dosis von der Tablette 4 abbürstet.

Durch den Inhalationsvorgang strömt nach Öffnung der Klappe 17 Luft durch die Lufteinlaßschlitze 20 am Boden 19 des Gehäuses nach, welche die abgebürsteten Partikel zum Mundstück 16 transportiert.

Nach Beendigung des Inhalationsvorganges wird die nicht dargestellte Schutzkappe aufgesetzt. Beim Aufsetzen wird der Tablettenhalter 1 auf den Geräteboden 19 gedrückt und die Tablette 3 von der Bürste 5 getrennt. Dies dient der Entlastung der Borsten.

Der flanschartige Teil des Tablettenhalters 1 dient (nach Abnehmen der Schutzkappe) gleichzeitig als Verbrauchsanzeiger, da sich seine Position entsprechend dem Tablettenverbrauch verändert.

Die in Fig. 1 (und 2) dargestellte Ausführungsform des erfindungsgemäßen treibgasfreien Inhalationsgerätes läßt mehrere Varianten hinsichtlich seiner Baugruppen und Bauelemente zu.

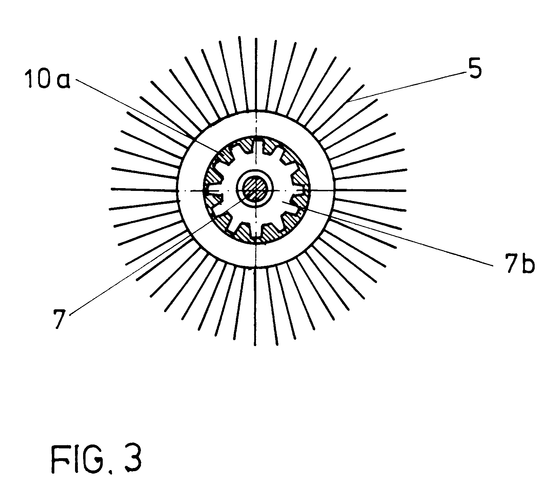

So kann das vom Bürstenrad 10 abtrennbare Antriebs-Reibrad 9 in Verbindung mit der Tellerfeder 8 durch einen Sperrklinkenmechanismus ersetzt werden, der die Spindel 7 und das Bürstenrad 10 beim Spannen der Feder entkoppelt. Ein derartiger Sperrklinkenmechanismus ist im Schnitt in der Fig. 3 dargestellt. Er weist ein geteiltes Antriebsritzel 7b auf, das mit Vertiefungen im Bürstenträger 10a in Antriebsverbindung kommt. Die Verbindung ist durch Entklinkung des Antriebsritzels lösbar.

Die in Fig. 1 dargestellte Bürste mit radialen Borsten und horizontaler Welle kann auch durch eine Topfbürste mit vertikaler Welle ersetzt werden, wobei deren Antrieb durch eine mit der Drucktaste 6 verbundene Zahnstange erfolgen kann.

Der die selbsttätige Auslösung des gespannten Zustandes beim aktiven Einatmen bewirkende Mechanismus mit der Klappe 17 und dem Kniehebelmechanismus 18 kann beispielsweise durch eine auf Unterdruck ansprechende Trigger-Kolbenanordnung ersetzt werden.

Auch die Lufteintrittschlitze 20 müssen nicht zwingend am Gehäuseboden vorgesehen sein, sondern sie können auch seitlich am Gehäuse 24 angeordnet werden. Zweckmäßig werden dabei im Gehäuseinneren entsprechende Luftführungswege geschaffen, die ein vollständiges, möglichst wandfreies Hinleiten der abgeriebenen Partikel zum Mundstück 16 gewährleisten.

Die Einheit, bestehend aus Tablette 4, Tablettenhalter 1, Tablettensockel 21, Druckfeder 2 und Schraubboden 22 kann aus dem Gerät herausgeschraubt und durch eine neue Einheit ersetzt werden. Die Tablettenführung 23 kann ggf. in die Wechseleinheit einbezogen werden, ebenso wie ggf. die Spindel 13 und der Anschlag 3.

Es ist auch denkbar, nur die Tablette 4 zu ersetzen.

Im übrigen muß das Mundstück 16 nicht kopfseitig am Gehäuse 24 angeordnet sein. Es ist ebensogut möglich, das Mundstück seitlich, bzw. in Höhe der Bürste 5, anzuordnen.

## Patentansprüche

1. Treibgasfreies Inhalationsgerät, mit einem Vorrat an zu inhalierender medizinischer Substanz (4), einer manuell betätigbaren Dosiereinrichtung (5) zur Entnahme einer vorgegebenen Dosis der medizinischen Substanz (4) für den jeweiligen Inhalationsvorgang, einem Mundstück (16) zum aktiven Einatmen, einem Luftraum zur Verteilung der jeweiligen Dosis der medizinischen Substanz (4) im Luftstrom und einer Halterung (1), in welcher der Vorrat an medizinischer Substanz (4), aufgenommen ist, dadurch gekennzeichnet, daß die medizinische Substanz verfestigt in einer vorgegebenen geometrischen Struktur (4) vorliegt und daß ihr als Dosiereinrichtung eine Abriebanordnung in Form einer drehbaren Bürstenanordnung (5) zum mechanischen Abrieb der vorgegebenen Dosis zugeordnet ist.

2. Inhalationsgerät nach Anspruch 1, mit einer verschiebbaren Halterung, dadurch gekennzeichnet, daß der Halterung (1), die gegen die Kraft einer Feder (2) frei verschiebbar angeordnet ist, ein einstellbarer Anschlag (3) zugeordnet ist, der den Verschiebeweg der Halterung begrenzt und die Dosis vorgibt.

3. Inhalationsgerät nach Anspruch 1 oder 2, dadurch gekennzeichnet, daß der Bürstenanordnung (5) ein Antrieb, vorzugsweise ein Federantrieb (9, 10, 11, 12), zugeordnet ist, der mittels eines Druckknopfes (6) manuell spannbar ist und der beim aktiven Einatmen auslösbar ist.

4. Inhalationsgerät nach Anspruch 3, dadurch gekennzeichnet, daß eine Schalteinrichtung (15, 17, 18) vorgesehen ist, die auf den Unterdruck im Luftraum des Mundstückes beim aktiven Einatmen anspricht und den Antrieb der Bürstenanordnung selbsttätig auslöst.

5. Inhalationsgerät nach Anspruch 4, dadurch gekennzeichnet, daß die Schalteinrichtung eine federvorgespannte Klappe (17) aufweist, die sich im Luftraum quer zum Luftstrom erstreckt und der ein Kniehebelmechanismus (18) zugeordnet ist, die mit einer Entklinkungsanordnung (15, 15a, 14) des Antriebes der Bürstenanordnung in Wirkverbindung steht.

6. Inhalationsgerät nach einem der Ansprüche 3 - 5, dadurch gekennzeichnet, daß der Federantrieb eine Spindel (7) aufweist, die auf der einen Seite mit dem Druckknopf (6) fest verbunden ist sowie die auf der gegenüberliegenden Seite an der Gehäusewand (24) des Inhalationsgerätes gelagert ist, die von einer durch translatorische Spindelbewegung spannbaren Antriebsfeder (11) umgeben ist und auf der, trennbar beim Spannen der Antriebsfeder, ein Antriebsrad (9) für die Bürstenanordnung (10, 5) zugeordnet ist.

7. Inhalationsgerät nach Anspruch 6, dadurch gekennzeichnet, daß das Antriebsrad ein Reibrad (9) ist, das mit einem weiteren Reibrad (10) in der Bürstenanordnung in Wirkverbindung steht, die durch translatorische Verschiebung der Spindel gegen die Kraft einer Feder (8) lösbar ist.

8. Inhalationsgerät nach Anspruch 6, dadurch gekennzeichnet, daß das Antriebsrad mit der Bürstenanordnung über eine Verklinkung, die durch translatorische Verschiebung der Spindel gegen die Kraft einer Feder lösbar ist, in Antriebs-Wirkverbindung steht (Fig. 3).

9. Inhalationsgerät nach einem der Ansprüche 1 - 8, dadurch gekennzeichnet, daß nach dem Inhalationsvorgang beim Übergang in den Nichtgebrauchszustand des Gerätes die Bürstenanordnung von der verfestigten Struktur der medizinischen Substanz trennbar ist.

10. Inhalationsgerät nach einem der Ansprüche 1 - 9, dadurch gekennzeichnet, daß die verfestigte Struktur der medizinischen Substanz die Form einer quaderförmigen Tablette hat.

## Claims

1. Inhalation device free from propellent gas, having a supply of medicinal substance (4) to be inhaled, a manually operable metering device (5) for removing a given dose of the medicinal substance (4) for the particular inhalation process, a mouthpiece (16) for active breathing in, an air space for distribution of the dose of medicinal substance (4) in the air stream and a holder (1) in which the supply of medicinal substance (4) is contained, characterised in that the medicinal substance is solidified in a given geometric structure (4), and is associated with the metering device constituted by an abrasion device in the form of a rotary brush arrangement (5) for mechanically abrading the given dose.

2. Inhalation device according to claim 1, having a movable holder, characterised in that the holder (1), which is mounted to be freely movable counter to the force of a spring (2), is associated with an adjustable stop (3) which limits the travel of the holder and predetermines the dose.

3. Inhalation device according to claim 1 or 2, characterised in that a drive, preferably a spring drive (9, 10, 11, 12) is associated with the brush arrangement (5) and is manually tensionable by means of a push button (6) and can be actuated by actively breathing in.

4. Inhalation device according to claim 3, characterised in that switching means (15, 17, 18) are provided which respond to the underpressure in the air space of the mouthpiece during active breathing in and automatically trigger the drive of the brush arrangement.

5. Inhalation device according to claim 4, characterised in that the switching means comprise a spring-biased flap (17) which extends at right angles to the air stream in the air space and which is associated with a bent lever mechanism (18) which is operatively connected to a discharging device (15, 15a, 14) for the drive of the brush arrangement.

6. Inhalation device according to one of claims 3 to 5, characterised in that the spring drive has a spindle (7) which is fixedly connected on the one hand to the push button (6) and which is mounted at the other end on the housing wall (24) of the inhalation device, which is surrounded by a drive springs (11) tensionable by a translatory spindle movement and on which a drive-wheel (9) for the brush arrangement (10, 5) is arranged, in such a way as to be separable when the drive spring is tensioned.

7. Inhalation device according to claim 6, characterised in that the drive-wheel is a friction wheel (9) which is operatively connected to another friction wheel (10) in the brush arrangement, and which is releasable by translatory movement of the spindle counter to the force of a spring (8).

8. Inhalation device according to claim 6, characterised in that the drive-wheel is operatively connected in driving mode to the brush arrangement via a latching mechanism which is releasable by translatory movement of the spindle counter to the force of a spring (Fig. 3).

9. Inhalation device according to one of claims 1 to 8, characterised in that, after the inhalation process, on moving into the position of non-use of the device, the brush arrangement can be separated from the solidified structure of the medicinal substance.

10. Inhalation device according to one of claims 1 to 9, characterised in that the solidified structure of the medicinal substance is in the form of a square tablet.

## Revendications

1. Inhalateur sans gaz propulseur, comprenant une réserve de substance médicamenteuse à inhaler (4), un doseur (5) qui peut être actionné manuellement afin de prélever une dose prédéterminée de la substance médicamenteuse (4) pour le processus d'inhalation correspondant, une embouchure (16) permettant une inhalation active, un espace d'air pour la dispersion de la dose correspondante de la substance médicamenteuse (4) dans un flux d'air et un support (1) dans lequel la réserve de substance médicamenteuse (4) est placée, caractérisé en ce que la substance médicamenteuse est solidifiée sous une forme géométrique (4) prédéterminée et en ce qu'à titre de doseur y est associé un dispositif d'abrasion ayant la forme d'un dispositif à brosses (5) rotatif destiné à produire par abrasion mécanique la dose prédéterminée.

2. Inhalateur suivant la revendication 1, comprenant un support coulissant, caractérisé en ce que le support (1), qui est monté de manière à pouvoir coulisser librement à l'encontre de la sollicitation d'un ressort (2) comporte une butée (3) réglable, qui limite la course du support et prédétermine la dose.

3. Inhalateur suivant la revendication 1 ou 2, caractérisé en ce que le dispositif à brosses (5) comporte un entraînement, de préférence, un entraînement à ressort (9, 10, 11, 12), qui peut être mis sous tension manuellement au moyen d'un bouton-poussoir (6) et qui peut être déclenché lors d'une inhalation active.

4. Inhalateur suivant la revendication 3, caractérisé en ce qu'un dispositif de déclenchement (15, 17, 18) est prévu, qui réagit à la dépression créée dans l'espace d'air de l'embouchure par l'inhalation active et déclenche automatiquement l'entraînement du dispositif à brosses.

5. Inhalateur suivant la revendication 4, caractérisé en ce que le dispositif de déclenchement comporte un clapet (17) sollicité par un ressort, qui s'étend dans l'espace d'air perpendiculairement au flux d'air et qui comporte un mécanisme à genouillère (18), qui se trouve en liaison active avec un dispositif de décliquetage (15, 15a, 14) de l'entraînement du dispositif à brosses.

6. Inhalateur suivant l'une quelconque des revendications 3 à 5, caractérisé en ce que l'entraînement par ressort comporte un arbre (7), qui, d'un côté, est relié rigidement au bouton-poussoir (6) et, de l'autre côté, tourillonne dans un logement sur la paroi du boîtier (24) de l'inhalateur, qui est entouré par un ressort d'entraînement (11) pouvant être comprimé par translation de l'arbre et sur lequel est monté une roue d'entraînement (9) pour le dispositif à brosses (10, 5), qui peut être désaccouplée lors de la compression du ressort d'entraînement.

7. Inhalateur suivant la revendication 6, caractérisé en ce que la roue d'entraînement est une roue de friction (9), qui est en liaison active avec une autre roue de friction (10) du dispositif à brosses, cette liaison pouvant être interrompue par translation de l'arbre à l'encontre de la sollicitation d'un ressort (8).

8. Inhalateur suivant la revendication 6, caractérisé en ce que la roue d'entraînement est en liaison active d'entraînement avec le dispositif à brosses au moyen d'un verrouillage, qui peut être libéré par translation de l'arbre à l'encontre de la sollicitation d'un ressort (Fig. 3).

9. Inhalateur suivant l'une quelconque des revendications 1 à 8, caractérisé en ce que, après le processus d'inhalation, au passage à l'état de repos de l'appareil, le dispositif à brosses peut être séparé de la structure solidifiée de la substance médicamenteuse.

10. Inhalateur suivant l'une quelconque des revendications 1 à 9, caractérisé en ce que la structure solidifiée de la substance médicamenteuse a la forme d'un comprimé parallélépipédique.
